Europäisches Patentamt

**European Patent Office**

**Office européen des brevets**

(11) Publication number: **0 133 608**
**A1**

## EUROPEAN PATENT APPLICATION

(21) Application number: **84830041.4**

(22) Date of filing: **17.02.84**

(51) Int. Cl.⁴: **A 61 F 2/24**

(30) Priority: **12.04.83 IT 4808183**

(43) Date of publication of application:
**27.02.85 Bulletin 85/9**

(84) Designated Contracting States:
**AT BE CH DE FR GB LI NL SE**

(71) Applicant: **Marconi, Valter**
**Via A. Barilatti n. 72**
**I-00144 Roma(IT)**

(72) Inventor: **Marconi, Valter**
**Via A. Barilatti n. 72**
**I-00144 Roma(IT)**

(54) **Double-leaflet cardiac valvular prosthesis.**

(57) Cardiac valvular prosthesis of mechanical type not requiring for the opening and closing movement of the leaflets (2) the presence of hinges or pins. The leaflets (2) open and close, with rotational and translational movement, by slipping along curved tracks (3), (two per each leaflet (2)) made out in the inside of the housing ring (1) of the valve, in its central position, and jutting out of the ring (1). In location fit with these jutting out slipping tracks (3), the side edge of the leaflets (2) is provided with suitable notches or indentations aiming both at making the leaflet (2) rotate and at preventing its coming out of the valve housing.

FIG. 2ª

FIG 2^b

SEZ."A-A"

FIG 2^c

FIG 2^d

Description

This invention relates to a cardiac valvular prosthesis of mechanical type, provided with two leaflets.

A mechanical valvular prosthesis generally consists of

a) a circular flow orifice

b) the flow orifice occluder, such as a disc, a ball, or two leaflets, generally semicircular

c) structures aiming both at correctly guiding the occluder, and at preventing it from malpositioning or from coming out of the valve housing.

These structures have been to date realized by two ways:

1) by constraining the occluder (consisting of discs or leaflets) to the valve housing, by means of pins or hinges of different type

2) by constraining the occluder, consisting of a ball, a disc, or leaflets, by means of structures consisting of wire, cages or metallic rings.

The following valves, for example, belong to the first category: St. Jude Medical (Emery et al., Circulation, 58, (Suppl. 11), 83, 1977); Huffstutler et al., U.S. Pat n. 4.240.161, issued on 23rd, Dec., 1980; Bloch, U.S. Pat. n. 3,953,898, 4th, May, 1976; Wada, U.S. Pat. n. 3,445, 863, 27th May, 1969; Klawitter, U.S. Pat. n. 4,308,624, 5th June, 1982; Mc Leod, U.S. Pat. n. 3,926,215, December, 1975.

Valves of the second type are instead, besides the wellknown Starr-Edwards valve (ref. A. Starr and M.L. Edwards, Annals of Surgery 154, 726, 1961), the Smeloff – Cutter valve (ref. M.M. Mc Henry et al., Circulation 51, 52: Suppl. 2 : 215, 1975), the Biork-Shiley valve (ref. V.O. Biork et al., Scandinavian J. Thoracic Cardiovascular Surgery 5, 177-191, 1971) and the Lillehei-Kaster, valve (ref. R.L. Kaster et al., transactions Amer. Soc. Artificial Internal Organs 16 : 232-243, 1970), the ones described by Hall (K.V. Hall et al., J. Oslo City Hospital, 29, 3-21, 1979), by Kaster (U.S. Pat. n. 3,959,827, 1st June 1976) by Marconi (Italian Patent Applications nos. 48040 A/81 and 49893 A/81).

These abovementioned valves are often characterized by a complex structure involving construction difficulties and, even if they can often be considered as remarkable improvements in the history of cardiac prosthetic valves, are not free from use limitations and from possible complications related to their design. The pins and hinges existing in the valves of the abovementioned type 1) can be dangerous starting points of blood clots (L. Nunez et al., Annals Thoracic Surgery, 29, no. 6, 567, 1980), and also wire structures can be, although at a lower degree, considered responsible for clot formation.

Moreover these structures disturb, often in a remarkable amount, the necessary centrality and laminarity of the blood flow. The construction of these structures often is also difficult and expensive, and furthermore possible welding defects can cause the detachment of wire from the ring or its breakage, with catastrophic results for the patient. It turns out that a new mechanical prosthesis being able to satisfy the numerous requirements of a cardiac valve and not requiring for its realization either the presence of pins or hinges, or that of wire structures, would represent a real technical progress. Such a prosthesis would indeed guarantee both better performance and simpler and cheaper execution.

The present invention precisely relates to a cardiac valvular prosthesis of mechanical type provided with these features, which can be obtained from a suitably shaped metallic ring, with remarkable advantages such as simple and more precise execution, lower cost and absence of weldings.

This valve consists of a circular orifice (or housing ring) containing two roughly semi-circular leaflets. These leaflets can be either straight or provided with a slight curvature.

Each leaflet, owing to the blood flow, performs a rotational and translational movement, by slipping along two opposite tracks, whose curvature compels the leaflet to perform the correct movement of opening and closing. Moreover, these tracks constitute a constraint which the leaflets cannot come out of.

These tracks jutting out of the housing ring are located in its internal part, in central position, and form with the ring one continuous structure.

They can have, besides the circular section, section of different type, such as triangular, square, rectangular and so on.

In location fit with these slipping tracks jutting out of the internal part of the flow orifice, the side edges of the leaflets (the leaflets are also located inside the flow orifice) are provided with suitable notches, or indentations, aiming both at making the leaflet rotate and at preventing its coming out of the valve housing, particularly when, at the end of the opening phase, the leaflets are almost perpendicular to the plane of the housing ring.

Since the tracks internal to the housing ring and the notches on the edge of the leaflet are complementary, the profile of the notch will preferably be similar to the type of section possessed by the track (i.e. semicircular or triangular or rectangular and so on). This complementarity between leaflet and slipping track provides a system which is "self-cleaning" with respect to thrombi that can form on these surfaces, and this is an important technical improvement of the present invention.

The opening movement of the leaflet can be facilitated by pawls placed just above the leaflet, and aiming at making its quick rotation easy. The housing ring contains in its internal part, immediately below the leaflets when they are in the closure position, protuberances extending at least partially along the circumference of the housing ring. These protuberances, by causing a slight restriction of the diameter of the housing ring, permit a good support to the leaflets in the closure position, and guarantee in this phase the absence of blood regurgitation.

The leaflets' stop at the end of the opening phase is guaranteed by short and strong metal structures jutting out about of the upper rim of the housing ring and turned towards its internal part.

These metal structures jutting out of the internal part of the housing ring can be either inclined towards the lower rim of the ring and tight to its internal surface, or oriented upward, and can be joined each other to form one metal structure.

Fig. 1a shows the side picture of a valve where 1 represents the housing ring, 3 the slipping tracks jutting out of the internal part of the ring and 5 represents the protuberance on which the leaflets edge lean when the leaflets are closed.

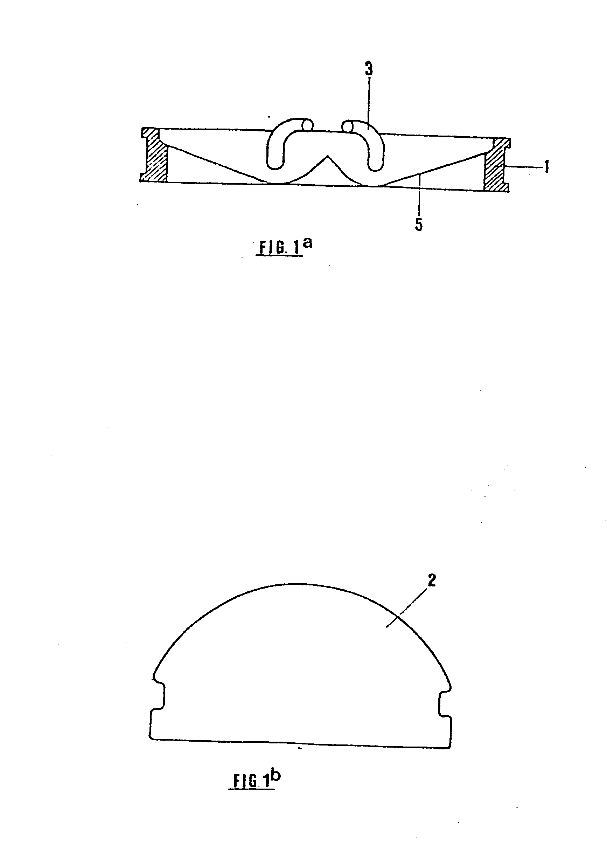

Fig. 1b shows a leaflet provided, on its side edge, with the notches that have to interact with the slipping tracks.

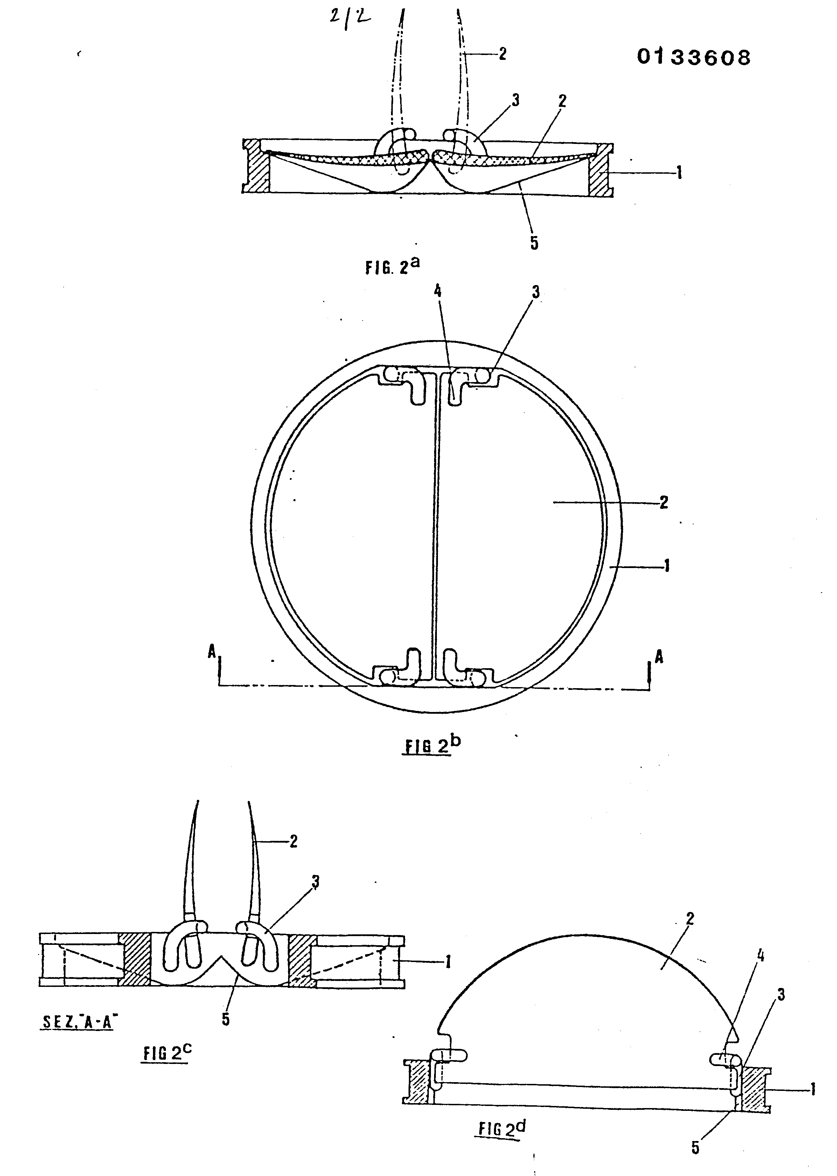

Fig. 2a shows a side picture where the leaflets (reference number 2) are represented both in the opening (dashed) and in the closing position.

Fig. 2b shows the horizontal projection of the same realization, where 4 represents the nibs stopping the leaflets in their maximum opening position.

Fig. 2c, representing a section of Fig. 2b, illustrates the interaction of the leaflets (here shown in their maximum opening position) with

-5-

the slipping and stop structures.

Fig. 2d represents another side projection showing the leaflets in their maximum opening position.

In all figures the reference numbers have the abovementioned mean=
ing.

What we claim is:

1) A cardiac valvular prosthesis of mechanical type not containing any structure such as pins, hinges or metallic filaments, and mainly consisting of:

a) two nearly semi-circular or ellipsoidal leaflets

b) a housing ring containing in its internal part, per each leaflet, two curved tracks, opposite and jutting out of the ring, and extending from the basis as far as the upper rim of the ring.

c) structures able to stop the leaflets at the end of their opening phase.

Each leaflet being provided, on its side edge, with two notches (or indentations) that are in location fit with the slipping tracks along which the leaflets open and close by a translational and rotational movement.

2) Valvular prosthesis according to claim no. 1 characterized in that it possesses, below the leaflets and extending at least along a part of the circumference of the housing ring, in its internal part, a protuberance aiming both at supporting the leaflets and at avoiding blood regurgitation when the valve is closed.

3) Valvular prosthesis according to clains 1) and 2) characterized in that the section of the slipping tracks of the leaflets can be, besides circular, triangular, square, or rectangular

4) Valvular prosthesis according to the previous claims characterized in that the stop of the leaflets at the end of their opening phase is guaranteed by metallic structures tight to the housing ring and projecting towards its internal part.

5) Valvular prosthesis according to the previous claims characterized in that, in order to facilitate the rotational and translational

-2-

movement of the leaflets, the ring possesses in its inside some pawls (two per each leaflet) placed immediately above the leaflet.

6) Cardiac valvular prosthesis of mechanical type as substantially described and pictured in the figures.

3

1

5

FIG. 1ᵃ

2

FIG. 1ᵇ

0133608

FIG. 2ᵃ

FIG 2ᵇ

SEZ. "A-A"

FIG 2ᶜ

FIG 2ᵈ

0133608

Application number

# EUROPEAN SEARCH REPORT

## DOCUMENTS CONSIDERED TO BE RELEVANT

EP 84830041.4

| Category | Citation of document with indication, where appropriate, of relevant passages | Relevant to claim | CLASSIFICATION OF THE APPLICATION (Int. Cl. ³) |
|---|---|---|---|
| Y<br>A | US - A - 3 538 514 (G. SCHIMERT)<br>* Totality * | 1-3,5<br>4 | A 61 F 2/24 |
| Y<br>A | EP - A2 - 0 050 971 (HEMEX INC.)<br>* Totality, especially fig. 1-5, page 6, lines 9-18 * | 1-3,5<br>4 | |
| A,D | EP - A1 - 0 061 430 (V. MARCONI)<br>* Abstract * | 1,4 | |

|  |
|---|
| **TECHNICAL FIELDS SEARCHED (Int. Cl. ³)** |
| A 61 F |

The present search report has been drawn up for all claims

| Place of search | Date of completion of the search | Examiner |
|---|---|---|
| VIENNA | 19-07-1984 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82